(19) Europäisches Patentamt
European Patent Office
Office européen des brevets

(11) Publication number: **0 085 031 B1**

(12) # EUROPEAN PATENT SPECIFICATION

(45) Date of publication of the patent specification: **25.06.86**

(21) Application number: **83830005.1**

(22) Date of filing: **19.01.83**

(51) Int. Cl.⁴: **C 07 C 49/84,** C 07 C 49/813, C 07 C 43/307, C 07 C 41/52, C 07 C 45/29, C 07 C 45/61, C 07 C 45/63, C 07 C 41/56, C 07 C 41/50, C 07 C 41/48, C 07 D 229/02

(54) **Method for the preparation of symmetrical monoacetals of aromatic 1,2-diketones starting from the corresponding alphahydroxyketones.**

(30) Priority: **22.01.82 IT 4763182**

(43) Date of publication of application: **03.08.83 Bulletin 83/31**

(45) Publication of the grant of the patent: **25.06.86 Bulletin 86/26**

(84) Designated Contracting States: **DE FR GB**

(56) References cited:
**DE - A - 2 337 813**
**DE - A - 2 616 382**
**DE - A - 2 616 588**
**DE - B - 1 254 138**

**Patent Abstracts of Japan vol. 6, no. 3, 9 January 1982**
**THE JOURNAL OF ORGANIC CHEMISTRY, vol. 45, no. 14, 1980, Columbus, Ohio (US); N.De Kimpe et al.: "Rearrangement of 1-aryl-2,2-dihalo-1-alkanones", pp. 2803-2813**
**CHEMISCHE BERICHTE, vol. 94, no. 8, 1961, Weinheim/Bergstr. (DE); R.Kuhn et al.: "Monoketale von 1,2-Diketonen", pp. 2258-2263**

(73) Proprietor: **F.LLI LAMBERTI S.p.A., Via Piave 18, I-21041 Albizzate (VA) (IT)**

(72) Inventor: **Li Bassi, Giuseppe, Via Preja 4, Gavirate (Varese) (IT)**
Inventor: **Cadona', Luciano, Via Tatto 11, Varese (IT)**

(74) Representative: **Moriconi, Franco, Via Mario Fani 37, I-00135 Roma (IT)**

EP 0 085 031 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a new method for the production of the symmetrical monoacetals of 1,2-aromatic diketones starting from the corresponding alphahydroxyketones.

It is well known to people skilled in the art that mixtures of unsaturated monomers such as acrylic, vinylic, allylic, maleic, etc... and of unsaturated oligomers or polymers can be polymerised by irradiation with ultraviolet light in presence of chemical products which, under its influence, are able to generate radicals. Such mixtures can be employed advantageously in various fields of application, such as varnishes for wood and plastic materials, paper and carton coating, metal painting and preparation of inks and adhesives.

Products able to induce the polymerization of unsaturated molecules under the influence of light are then called photoinitiators of polymerization and some of them have become industrially very interesting, because they have quantum yield characteristics (rate of polymerization for unit of absorbed energy), efficiency (lower amount of photoinitiator to obtain the desired characteristics), and dark storage stability not otherwise combined.

Particularly monoacetals of aromatic 1,2-diketones show all these advantages and therefore they have found pratical use in various application fields. Such type of products are already known in the scientific literature and in patents (i.e. U.S.P. 2.995.573 and U.S.P. 3.715.293) and moreover industrial processes for their production have been developed.

With regard to products like benzilmonoacetals, one of the proposed method (OLS 2.337.813) includes the acetalization of 1,2-aromatic diketones, possibly obtained by oxidation of the corresponding benzoins according to L.F. Fieser et al (J. Org. Chem. 27, 2247 (1962)), by means of dialkylsulfites in presence of acidic catalysts according to the findings of W. Woss (Annalen, 485, 283 (1931)) reported on Houben Weyl, Methoden der Organischen Chemie (Ed. 1965, Band VI/3, Sauerstoffverbindungen I. Teil 3 – Pag. 228).

A second method (OLS 2.616.588) includes the reaction between the same aromatic 1,2-diketones, an alkylating agent and an alkaline metal alcoholate, as an extension of the alkoxylation of tertiary carboxonium salts studied by E. Schmitz (Berichte, 91 410 (1958)) and reported on Houben Weyl, Methoden der organischen Chemie, (Ed. 1965) Band VI/3, Sauerstoffverbindungen I, Teil 3 – Pag. 263).

Another advantage of this method is reported by R. Kuhn et al, Chem. Ber. 94, 2258 (1961).

The advantage of this second method in comparison to the first one its the possibility to obtain asymmetric acetals.

The object of the present invention is to obtain noncyclic symmetrical monoacetals of aromatic 1,2-diketones, starting directly from the corresponding alphahydrxyketones, without any intermediate formation of the relative 1,2-diketones.

It has been found that noncyclic symmetrical monoacetals of aromatic 1,2-diketones having the formula

```
     O  OR
     ||  |
Ar1–C–C–Ar2                I
         |
         OR
```

wherein:

– $Ar_1$ and $Ar_2$ represent, each independently, radicals of carbocyclic or heterocyclic aromatic nuclei, equal or different, which may also bring substituents such as halogen alkoxyl (with 1 to 5 carbon atom chain), phenyl, alkyl;

– R represents methyl, alkyl (having 2 to 5 carbon atom chain), alkenyl from 2 to 3 carbon atoms, trimethylsilyl, or an alkyl bearing substituents of the type $-OR_1$, $-SR_1$, $NR_2R_3$ wherein $R_1$, $R_2$, $R_3$ represent, each independently, an alkyl from 1 to 3 carbon atoms;

can be obtained by a simple method, generally applicable, with high yield and degree of purity, starting from the coresponding aromatic alphahydroxyketones having the formulas

```
     O  OH
     ||  |                 II
Ar1–C–CH–Ar2

     OH  O
     |   ||                III
Ar1–CH–C–Ar2
```

wherein:

– $Ar_1$ and $Ar_2$, each independently, have the same meaning as in formula I, without any intermediate formation of the relative aromatic 1,2-diketones, of the alphahydroxyketones of formula II and III are reacted with suitable oxidizing agents, with or without the protection of the ketonic group according to the type of reagents to be employed.

When the carbonyl protection is not used, it is possible to obtain, by the action of halogenating agents as halogens or sulfuril halides, intermediates of formula IV, which, without being isolated, are converted into products of formula I by suitable nucleophyles as alkaline alkoxylates:

```
     O  X
     ||  |
Ar1–C–C–Ar2                IV
         |
         Y
```

wherein:

– $Ar_1$ and $Ar_2$ have the same meaning as in the formula I;

– X, Y may be:

– equal, and in such a case, they represent an halogen radical,

– different, and in such a case, they represent

an halogen and respectively, an alkoxyl radical of the type – OR, in which R have the same meaning as in formula I.

In the case in which the carbonyl group is protected by converting it into an hydrazone or a symmetrical aceta- or into other addition compounds containing halogens, products of the formula:

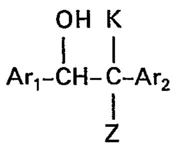

V

may be obtained, which, without being isolated, are oxidized by the methods well known to the people skilled in the art, to products of formula

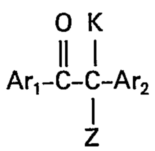

VI

which, without being isolated, are further acetalized, if the formula V does not represent itself an acetal, in order to obtain the products of formula I.

In the formulas V and IV:

$Ar_1$ and $Ar_2$ have the same meaning as in the formula I and K, Z may be:

- equal, and in such a case, they represent an halogen radical, alkoxyl
- OR (wherein R has the same meaning as in formula I);
- coincident, and in such a case, they represent a diazo or hydrazono group;
- diffferent, and in such a case, they represent an halogen radical and an alkoxyl – OR (wherein R has the sam meaning as in formula I) respectively.

Examples of aromatic alpha hydroxyketones are benzoin; symmetrical and asymmetric benzoins eventually substituted on the aromatic rings by alkyls, alkoxyls, phenyls, halogens; 2-thenoin; 2-furoin; 2-pyridoin; 1-(2-pyridyl)-2-phenyl-2-hydroxyethanone; 1-phenyl-2-(2-pyridyl)-2-hydroxyethanone; etc., and they are obtained by methods easily accessible in the literature.

The alphahydroxyketones preferred in this invention are those with symmetrical substituents such as benzoin, 2-thenoin, 2-furoin etc.

By the method described in this invention, it is therefore possible to produce the monoacetals of aromatic 1,2-diketones, described by the formula I.

Methods of Synthesis

The products of formula I can be obtained according to the present invention, through one of the following methods of synthesis:

Method A

It relates to the mono or dihalogenation on the carbon atom adjacent to the carbonyl position of an alphahydroxyketone by using halogen, sulfuryl halides, N-halogenoimides and further substitution of halogens by alkoxyl radicals.

a) When halogenation is performed in aprotic solvents, it is possible to obtain the germinal dihalides that are converted into products of formula I by treatment with alkaline alcoholates:

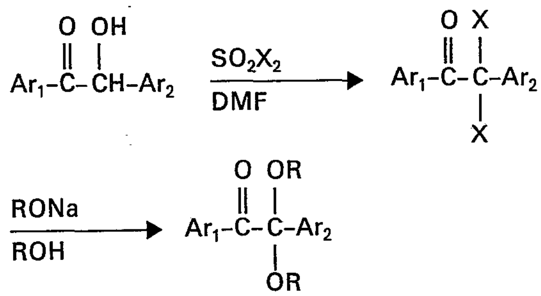


In this case, preferred starting compounds are those in which $Ar_1$ is the same as $Ar_2$, because in such way no separation of structural isomers is necessary, allowing high yields. The preferred reagents for the halogenation are sulfuryl chloride and chlorine, while the preferred alcoholates are sodium methylate or ethylate. The halogenation reaction proceeds with a paticularly high rate and according to a surprisingly different pathway, when compared with the results of L. Fieser et al (J. Org. Chem., 27 2247 (1962)), if an aliphatic amide, preferably N,N-dimethylformamide is employed as solvent. The subsequent exchange with alkaline alcoholates is not difficult and is performed in an alcoholic solvent. This reaction has already been studied on acetophenones by N. De Kimpe (J. Org. Chem. 43 2933 (1978) and Synth. Comm. 9 575 (1979)).

b) If the halogenation is performed in alcoholic solvent, an alphahalogeno-ether (hemiacetal halide) is obtained, which in presence of the same alcohol and an acid catalyst gives an acetal:

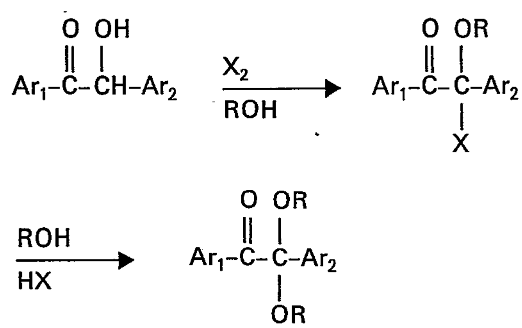


The alphachloroethers are known intermediates in the preparation of acetales, through the reaction of the diazoalcanes by alcohols and t-butylhypochlorite, as reported by H. Baganz, Angew. Chem. 78, 448 (1966)).

Also in this case the reaction conditions are smooth, yields are high and the products are isolated with high degree of purity.

Method B

According to this method, the carbonyl group is firstly protected by conversion into an acetal or an hydrazone and then the alcoholic group is oxidized to ketone.

a) Protection by acetalisation of the carbonyl. The alphahydroxyacetals are easily accessible through two reaction steps: firstly alphahydroxyketones are converted into monohalogenoketones by the action of a thionyl halide in presence of pyridine (A.M. Ward, J. Chem. Soc. 1541 (1929)) or more advantageously, be the action of a aqueous hydrogen halide, an organic solvent (which dissolves the product but is immiscible with water) and a phase transfer catalyst (D. Landini et al, Synthesis 430 (1975)).

In the second step the reaction of the alphahalogenoketones with two moles of an alkaline alcoholate gives, via an epoxyether, the alphahydroxyacetals:

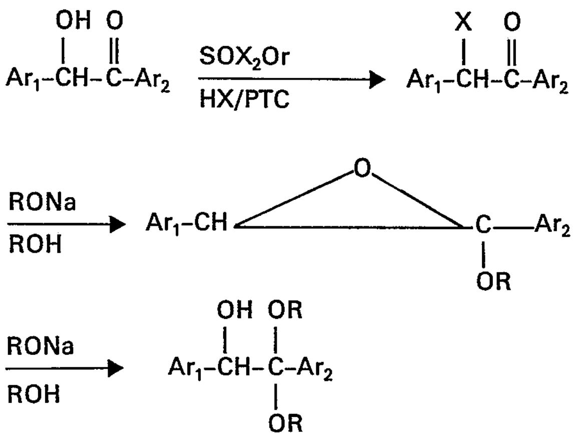


The further oxidation into compounds of formula I is performed according to classical methods by means of reagents that don't cleave the acetal group, as, for example, the Sarett reagent (F. Huet et al, Synth Commun. 10, 83 (1980)) or the Cornforth or the Snatzke reagent (Ber. 94, 729 (1961)), in an inert solvent at room temperature. Other reagents may be potassium permanganate (J. Am. Chem. Soc. 81, 633 (1959)) or potassium-2-nitropropane.

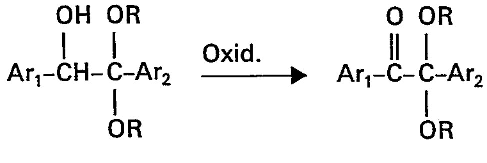


The oxidation may be also achieved with less traditional methods for instance, by means of the phase transfer catalysis. In such way sodium hypochlorite is used as oxidizing agent, the organic product is dissolved in methylene chloride and the phase transfer catalyst is a quaternary ammonium salt.

Some examples of oxidations carried on alcohols or benzylic type amines are already known in the literature (G.A. Lee, Te.Le. 20, 1641-1644 (1976)).

b) When the hydrazonic protection is used, firstly the alpha hydroxyketone is reacted by hydrazine under conditions that give the alphahydroxymonohydrazone derivative and this is subsequently oxidized giving an alphadiazoketone that, by further treatment by t-butilhypochlorite in alcoholic solvent, gives compounds of formula I:

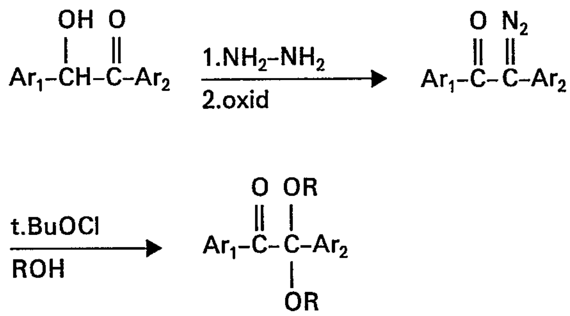


The oxidation of the hydrazono to the diazo group may be performed in homogeneus phase by an organic peracid (Belg. P. 802.112) or through phase transfer catalysis (J. R. Adamson et al, J. Chem. Soc. Perkin Trans. I, 2030 (1975)), separately or simultaneously to the oxidation of the hydroxyl group according to the method described in section B.a).

The conversion of the diazoketone into an acetal of the 1,2-diketone is very easily accomplished with high yields and gives very pure products; examples of such conversions are already known for aliphatic alphadiazoketones and for alphadiazoacetophenones (DBP 1.254.138). Alternatively the alphahydroxyketones may be obtained through the oxidation of the corresponding hydroxyhydrazones to hydroxydiazo derivatives, subsequent acetalization to alphahydroxyacetals and final oxidation of the hydroxyl to the ketonic group.

Furthermore and preferably, the oxidations of different oxidizable groups may be carried out simultaneously and the isolation of the alphadiazoketone is not required as a preliminary step in the production of the monoacetals of the 1,2-diketones of formula I.

Examples

Method A

Example 1

2,2-dichloro-1,2-diphenylethanone

6.6 g (90 mmol) of N,N-dimethylformamide are dropwise added to 50 ml (d = 1.67, 618 mmol) of stirred sulfurylchloride at 14-15°C, out of moisture.

While stirring 65 g (306 mmol) of benzoin are added portionwise and the reaction mixture is left without stirring and heated at 40-43°C for further 45 minutes. The mixture is chilled in ice and 21 g of glacial acetic acid diluted with 12.7 g of water is added followed by 35 g of water. The reaction product is extracted with toluene and the organic

layer washed with water and neutralized with sodium bicarbonate.

Evaporation of solvent under reduced pressure gives 73.8 g (91%) as crystalline solid. It coud be directly employed for the further reaction step.

A little amount is purified by silica gel column chromatography using petroleum ether/benzene (75/25 by weight) as eluent. After evaporation of the eluent, a white crystalline solid is obtained which melts at 59°C and has a chlorine content of 26.6% (calc. 26.8%).

The infrared spectrum shows characteristic bands at 1705 $cm^{-1}$ (C=O), 1220, 735 and 695 $cm^{-1}$.

Example 2

2,2-dimethoxy-1,2-diphenylethanone

73.8 g (0.278 mol) of the product obtained in the example 1 are dissolved in 500 ml of methanol and dropwise treated at 5–6°C with a solution of 37.6 g (0.696 mol) sodium methylate dissolved in 150 ml of methanol. The mixture is left at room temperature for 16 hours and then neutralized to pH 7–8 by acetic acid, the solid is filtered off and the solvent is removed under reduced pressure. The residue is taken up with 45.2 g of methylenchloride and is filtered yielding a solution containing 67.8 g (95%) of the title product.

A little amount of the solution is evaporated to dryness giving crystalline white solid that melts at 62–63°C and shows infrared absorption bands at 2940, 1695 (C=O) 1240, 1115, 1065, 700 $cm^{-1}$. A little amount is hydrolised in water by heating at 60–70°C with a trace of hydrochloric acid giving a yellow crystalline solid identified as benzil (2-keto-1,2-diphenylethanone m.p. 94–96°C) and 24.5 g of methanol per 100 g of analyzed product (calc. 25%).

Method B

Example 3

2,2-dimethoxy-1,2-diphenylethanol

106.1 g (0.5 mol) of benzoin are suspended in 53 g (0.67 mol) of pyridine and dropwise treated at 15–40°C with 79.5 g (0.66 mol) of thionylchloride.

After 1 hour the reaction mixture is cooled to 10°C and treated with 265 of ice giving a solid that is separated by filtration and dried under vacuum.

A little amount is characterized after crystallization from petroleum ether obtaining a white crystalline solid that melts at 67–68°C and shows infrared absorption bands at 1695 (C=O), 1450, 1210, 850, 735, 690 and 675, 545 $cm^{-1}$.

The solid is then dissolved in 460 ml of methanol and treated at 20–30°C with 81 g (1.5 mol) of sodium methylate dissolved in 325 ml of methanol.

After 2 hours at room temperature the mixture is neutralised to pH 7–8 by acetic acid, the solid is filtered off and the solvent removed under reduced pressure.

The residue weighs 122.7 g (95%); it is a viscous oil with little tendency to crystallization and shows infrared absorption bands at 2940, 1450, 1115, 1060, 700 $cm^{-1}$ and no carbonyl absorption.

Example 4

2,2-diemthoxy-1,2-diphenylethanone

The product obtained in the example 3 (122 g, 0.472 mol) is dissolved in 150 ml N,N-dimethylformamide and dropwise added to a solution of 70 g (0,70 mol) of chromic anhydride dissolved in 700 ml of N,N-dimethylformamide. The reaction mixture is cooled at 20°C and treated with 1 ml 98% of sulfuric acid.

After 16 hours the mixture is extracted with 300 ml of toluene and the organic layer washed with water and acqueous sodium bicarbonate.

After evaporation of the solvent the residue (109 g, 90%) is taken up with 163 g of styrene giving a 40% solution.

Example 5

2-diazo-1,2-diphenylethanone

3.2 g (100 mmol) of anhydrous hydrazine are added to a stirred suspension of 21.2 g (100 mmol) of benzoin in 100 ml of methylenechloride. After treating at 60–80°C for 30 minutes the reaction mixture is chilled and diluted with 100 ml of water. An addition is made under efficient stirring of 0.68 g (2 mmol) of tetrabutylammonium bisulfate, 2 ml 1% iodine solution in methylenechloride, and, slowly in 2 hours at 0–10°C, 17.7 ml (105 mmol) 38% peracetic acid, together with a 15% NaOH solution to control the pH at 9–10. After 2 hours 52 g (105 mmol) of 15% sodium hypochlorite solution is added. After further 2 hours of stirring the aqueous phase is discharged and the organic one is washed, dried on anhydrous sodium sulfate and the solvent removed under vacuum at 40°C max, yielding 17.8 g (80%) of the title compound.

A little amount of this product is crystallized from petroleum ether for the characterisation, giving a yellow crystalline solid that melts at 76–78°C and shows infrared absorption bands at 2070 (diazo), 1620 (C=O), 1360, 1240, 850, 755, 710, 690, 640 $cm^{-1}$.

Example 6

2,2-dimethoxy-1,2-diphenylethanone

22.2 g (100 mmol) of the product obtained in the example 5 are dissolved in 100 ml of methanol and dropwise treated under stirring at −15°C with 11.95 g (110 mmol) t-butylhypochlorite.

After stirring for 30 minutes (meanwhile the temperature rises to 0°C) the reaction mixture is neutralized with 5.9 g (110 mmol) of sodium methylate dissolved in 20 ml of methanol.

The solid is filtered off and the solvent evaporated under reduced pressure, yielding 25.1 g (98%) of the title compound as a white crystalline solid with the same characteristics reported for the product obtained in the example 2.

Example 7

2,2-diethoxy-1,2-diphenylethanone

It is obtained by the same procedure described

in the example 6, substituting anhydrous ethanol for methanol.

The product shows m.p. at 60-61°C and infrared absorption bands at 1960 (C=O), 1230, 1110, 1050, 940, 760, 700 $cm^{-1}$.

**Claims:**

1. A process for the production of symmetrical monoacetals of aromatic 1,2-diketones of formula:

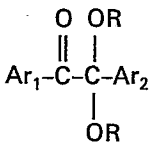

I

wherein

- $Ar_1$ and $Ar_2$ represent, each independently, radicals of carbocyclic or heterocyclic aromatic nuclei, equal or different, which may also bring substituents, as halogen, alkoxyl (with 1 to 5 carbon atom chain), phenyl, alkyl;
- R represents methyl, alkyl (with 2 to 5 carbon atom chain), alkenyl having 2 to 3 carbon atoms, trimethylsilyl, or an alkyl bearing substituents of the type $-OR_1$, $-SR_1$, $-NR_2R_3$ wherein, $R_1$, $R_2$, $R_3$ represent, each independently, an alkyl having 1 to 3 carbon atoms;

characterized by the fact that by reaction of the alpha-hydroxyketones of formulas:

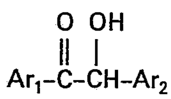

II

or

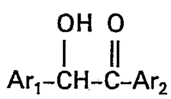

III

wherein:

- $Ar_1$ and $Ar_2$ have the aforementioned meaning, without protection of the carbonyl group, with halogenating agents, intermediates of formula:

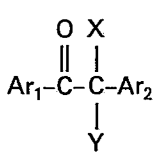

IV

wherein:

$Ar_1$ and $Ar_2$ have the same meaning as in formula I and X and Y may be:

- equal, and in such a case, they represent an halogen radical,
- different, and in this case they represent an halogen radical and, respectively, alkoxyl radical of the type -OR, wherein R has the same meaning as in formula I,

are obtained which are converted into products of formula I by nucleophyles, or starting from the aforesaid aromatic alpha-hydroxyketones, with protection of the carbonyl group by converting it into an hydrazone or an acetal, intermediates of formula:

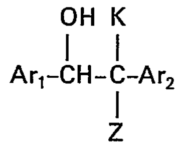

V

wherein:

$Ar_1$ and $Ar_2$ have the same meaning as in formula I and Z and K may be:

- equal, and, in such a case, they represent an halogen radical, alkoxyl -OR (wherein R has the same meaning as in formula I);
- coincident, and, in such a case, they represent a diazo or hydrazono group;
- different, and, in such a case, they represent an halogen radical and an alkoxyl of the type -OR (wherein R has the same meaning as in formula I), respectively,

are obtained which are transformed into products of formula I by oxidation.

2. A process according to claim 1 in which the intermediates of formula:

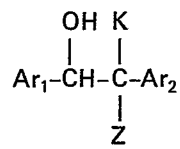

V

are oxidized and then acetalized.

3. A process according to claim 1 in which in the first step of the reaction an halogenating agent is employed together with a secondary aliphatic amide or an aliphatic alcohol having 1 to 5 carbon atoms, and in the second step an alkaline alcoholate on an alcohol is used.

4. A process according to claim 1 or 3 in which as halogenating agent, sulphurylchloride is employed in presence of N,N-dimethylformamide and, as alcoholate, sodium methylate or ethylate is used.

5. A process according to claim 1 in which, as oxidizing agents the Snatzke reagent (chromic anhydride in N,N-dimethyl-formamide) in methylene chloride, or organic peracids or sodium hypochlorite in mixed solvents (water-methylene chloride) in presence of phase transfer catalysts are used.

6. A process according to claims 1 and 2, in which the acetalization is carried out in methanol or ethanol in presence of t-butylhypochlorite.

**Revendications**

1. Procédé pour la production de monoacétals symétriques de 1,2-dicétones aromatiques de formule

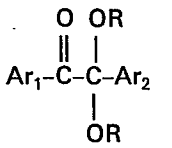

I

dans laquelle:

$Ar_1$ et $Ar_2$ représentent chacun, indépendamment l'un de l'autre, des radicaux de noyaux aromatiques carbocycliques ou hétérocycliques identiques ou différents qui peuvent également porter des substituants tels que halogène, alcoxyle (ayant une chaîne de 1 à 5 atomes de carbone), phényle et alkyle;

R représente un méthyle, un alkyle (ayant une chaîne de 2 à 5 atomes de carbone), un alcényle ayant 2 ou 3 atomes de carbone, un triméthylsilyle ou un alkyle portant des substituants du type $-OR_1$, $-SR_1$, $-NR_2R_3$ où $R_1$, $R_2$, $R_3$ représentent chacun indépendamment un alkyle ayant 1 à 3 atomes de carbone; caractérisé par le fait que par réaction des α-hydroxycétones de formules:

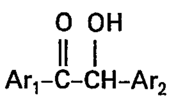

II

ou

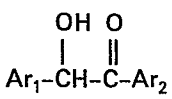

III

où

$Ar_1$ et $Ar_2$ ont la signification précédemment mentionnée, sans protection du groupe carbonyle, avec des agents d'halogénation, on obtient des intermédiaires de formule:

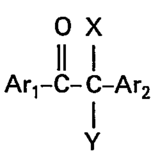

IV

dans laquelle:

$Ar_1$ et $Ar_2$ ont la même signification que dans la formule I et X et Y peuvent être:

– identiques, et dans ce cas ils représentent un radical halogène,

– différents, et dans ce cas ils représentent un radical halogène et respectivement un radical alcoxyle du type -OR, où R a la même signification que dans la formule I,

que l'on transforme en produits de formule I avec des nucléophiles, ou

à partir des α-hydroxycétones aromatiques précitées, avec protection du groupe carbonyle par transformation de celui-ci en une hydrazone ou un acétal, on obtient des intermédiaires de formule:

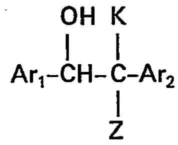

V

dans laquelle:

$Ar_1$ et $Ar_2$ ont la même signification que dans la formule I et Z et K peuvent être:

– identiques, et dans ce cas ils représentent un radical halogène, un alcoxyle –OR (où R a la même signification que dans la formule I);

– coïncidents, et dans ce cas ils représentent un groupe diazo ou hydrazono;

– différents, et dans ce cas ils représentent respectivement un radical holgène et un alcoxyle du type –OR (où R a la même signification que dans la formule I),

que l'on transforme en produits de formule I par oxydation.

2. Procédé selon la revendication 1 dans lequel on oxyde puis acétalise les intermédiaires de formule:

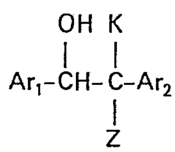

V

3. Procédé selon la revendication 1 dans lequel, dans le premier stade de réaction, on emploie un agent d'halogénation avec un amide aliphatique secondaire ou un alcool aliphatique ayant 1 à 5 atomes de carbone et, dans le second stade, on utilise un alcoolate alcalin dans un alcoól.

4. Procédé selon la revendication 1 ou 3 dans lequel, comme agent d'halogénation, on emploie le chlorure de sulfuryle en présence de N,N-diméthylformamide et, comme alcoolate, on utilise le méthylate ou l'éthylate de sodium.

5. Procédé selon la revendication 1 dans lequel, comme agents oxydants, on utilise le réactif de Snatzke (anhydride chromique dans le N,N-diméthylformamide) dans le chlorure de méthylène ou des peracides organiques ou l'hypochlorite de sodium dans des solvants mixtes (eau-chlorure de méthylène) en présence de catalyseurs de transfert de phase.

6. Procédé selon les revendications 1 et 2 dans lequel l'acétalisation est effectuée dans le méthanol ou l'éthanol en présence d'hypochlorite de tert.-butyle.

**Patentansprüche**

1. Verfahren zur Herstellung von symmetrischen Monoacetalen von aromatischen 1,2-Diketonen der Formel

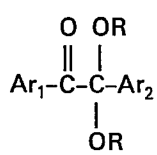

I

in der

- $Ar_1$ und $Ar_2$ jeweils unabhängig Reste von carbocyclischen oder heterocyclischen aromatischen Kernen bedeuten, die gleich oder verschieden sein können und auch Substituenten aufweisen können, wie Halogenatome oder Alkoxy- (mit 1 bis 5 Kohlenstoffatomen in der Kette), Phenyl- oder Alkylreste;

- R einen Methyl-, Alkyl- (mit 2 bis 5 Kohlenstoffatomen in der Kette), Alkenyl- mit 2 bis 3 Kohlenstoffatomen, Trimethylsilyl- oder Alkylrest mit Substituenten der Art $-OR_1$, $-SR_1$ oder $-NR_2R_3$ darstellt, wobei $R_1$, $R_2$ und $R_3$ jeweils unabhängig voneinander Alkylreste mit 1 bis 3 Kohlenstoffatomen bedeuten, dadurch gekennzeichnet, dass durch Umsetzung von α-Hydroxyketonen der Formeln

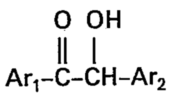

II

oder

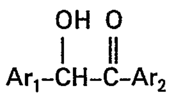

III

in denen

- $Ar_1$ und $Ar_2$ die vorstehend angegebene Bedeutung haben, mit Halogenierungsmitteln ohne Schutz der Carbonylgruppe Zwischenprodukte der Formel

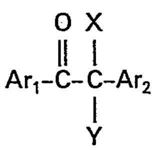

IV

in der

- $Ar_1$ und $Ar_2$ die gleiche Bedeutung wie in Formel I haben und X und Y

- gleich sein können, und in einem solchen Fall Halogenatome bedeuten, oder

- verschieden sein können, und in diesem Fall ein Halogenatom oder einen Alkoxyrest des Typs -OR bedeuten, in dem R die gleiche Bedeutung wie in Formel I hat,

erhalten werden, die durch nucleophile Verbindungen in Produkte der Formel I umgewandelt werden, oder ausgehend von den genannten aromatischen α-Hydroxyketonen mit Schutz der Carbonylgruppe durch ihre Umwandlung in ein Hydrazon oder Acetal Zwischenprodukte der Formel

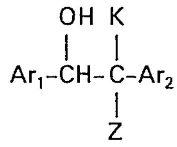

V

in der

$Ar_1$ und $Ar_2$ die gleiche Bedeutung wie in Formel I haben und Z und K

- gleich sein können, und in einem solchén Fall Halogenatome oder Alkoxyl -OR darstellen (wobei R die gleiche Bedeutung wie in Formel I hat)

- zusammengenommen sein können, und in einem solchen Fall eine Diazo- oder Hydrazonogruppe darstellen;

- verschieden sein können, und in einem solchen Fall ein Halogenatom und einen Alkoxyrest des Typs -OR darstellen (wobei R die gleiche Bedeutung wie in Formel I hat)

erhalten werden, die durch Oxidation in Produkte der Formel I umgewandelt werden.

2. Verfahren nach Anspruch 1, wobei die Zwischenprodukte der Formel

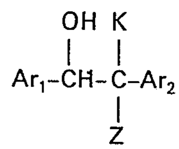

V

oxidiert und dann acetalisiert werden.

3. Ein Verfahren nach dem Anspruch 1, wobei in der ersten Stufe der Umsetzung ein Halogenierungsmittel zusammen mit einem sekundären aliphatischen Amid oder einem aliphatischen Alkohol mit 1 bis 5 Kohlenstoffatomen und in der zweiten Stufe ein alkalisches Alkoholat oder ein Alkohol verwendet werden.

4. Ein Verfahren nach den Ansprüchen 1 oder 3, wobei als Halogenierungsmittel Sulfurylchlorid in Gegenwart von N,N-Dimethylformamid und als Alkoholat Natriummethylat oder -äthylat verwendet werden.

5. Ein Verfahren nach dem Anspruch 1, wobei als Oxidationsmittel das Snatzke-Reagens (Chromsäureanhydrid in N,N-Dimethylformamid) in Methylenchlorid, oder organische Persäuren oder Natriumhypochlorit in Mischlösungsmitteln (Wasser-Methylenchlorid) in Gegenwart eines Phasenübertragungs-Katalysators verwendet werden.

6. Ein Verfahren nach den Ansprüchen 1 und 2, wobei die Acetalisierung in Methanol oder Äthanol in Gegenwart von t-Butylhypochlorid durchgeführt wird.